# FASCICULE DE BREVET EUROPEEN

(11) **EP 1 232 138 B1**
(45) Date de publication et mention de la délivrance du brevet: **19.10.2005**
(21) Numéro de dépôt: 00979736.6
(22) Date de dépôt: 15.11.2000
(51) Int. Cl.: C07C 57/075, C07C 67/54, C07C 69/54, C07C 51/44, C07C 67/62

(54) **PROCEDE DE PURIFICATION DES MONOMERES (METH) ACRYLIQUES PAR DISTILLATION**
VERFAHREN ZUM REINIGEN VON (METH)ACRYLMONOMEREN DURCH DESTILLATION
METHOD FOR PURIFYING (METH)ACRYLIC MONOMERS BY DISTILLATION

(30) Priorité: 24.11.1999 FR 9914777
(43) Date de publication de la demande: 21.08.2002
(73) Titulaire: Arkema, 92800 Puteaux (FR)
(72) Inventeur: FAUCONET, Michel, F-57730 Valmont (FR); LEPIZZERA, Stéphane, F-57500 Saint Avold (FR)
(74) Mandataire: Rieux, Michel
(86) Numéro de dépôt international: PCT/FR2000/003172
(87) Numéro de publication internationale: WO 2001/038285

(56) Documents cités:
- FR-A- 1 087 186
- FR-A- 2 056 825
- DATABASE WPI Week 8811 Derwent Publications Ltd., London, GB; AN 1988-073873 XP002142866 & JP 63 027457 A (MISUI TOATSU CHEM INC), 5 février 1988 (1988-02-05)

## Description

La présente invention porte sur un procédé de purification des monomères (méth)acryliques, tels que l'acide acrylique, par distillation.

Il est bien connu que l'un des points délicats de la purification ces monomères, en particulier de l'acide acrylique, provient de ce que ces monomères polymérisent facilement lorsqu'ils sont distillés.

La formation de polymères insolubles dans les équipements industriels de distillation entraîne alors des bouchages qui imposent un arrêt de l'installation et son nettoyage.

Pour pallier ces inconvénients, différents types de molécules stabilisantes sont employés. Cependant, ces stabilisants sont le plus souvent des molécules dont le point d'ébullition est nettement supérieur à celui du monomère. En conséquence, les vapeurs de monomère ne sont pas stabilisées et la condensation de celles-ci entraîne la présence de gouttelettes de monomère chaudes, liquides et non stabilisées. Dans la plupart des cas, ces gouttelettes viennent rapidement au contact d'un flux contenant des stabilisants. Cependant, dans la pratique industrielle, l'existence temporaire ou durable de points non mouillés par du liquide stabilisé est inévitable, et ces points de condensation constituent des points d'initiation et de propagation de polymérisation dans les colonnes à distiller.

Une des solutions pour parvenir à éviter la polymérisation de ces condensats non stabilisés consiste à injecter un gaz ayant des propriétés inhibitrices de polymérisation et permettant une distribution homogène dans la colonne. Le gaz NO présente de telles propriétés. En effet, cette molécule est un radical stable qui peut stopper la croissance des chaînes macromoléculaires et possède une tension de vapeur suffisamment élevée pour assurer une distribution homogène de celle-ci dans le milieu. Cependant, cette molécule réagit très rapidement avec l'oxygène pour former du NO₂, molécule décrite dans la littérature comme pouvant amorcer des polymérisations.

FR-A-1 567 710 décrit l'utilisation du gaz NO, en l'absence d'oxygène, lors de la synthèse de dérivés (méth)acryliques. FR-A-2 056 825 décrit l'utilisation du gaz NO, en l'absence d'oxygène, et de la phénothiazine (inhibiteur en phase liquide) durant la distillation de l'acide acrylique. EP-A-301 879 décrit l'utilisation d'un inhibiteur en phase liquide (couple composé phénolique/sel de Mn ou Ce) et d'un inhibiteur en phase gazeuse qui est soit du NO, soit le sel d'ammonium de la N-phényl N-nitroso-hydroxylamine, durant la distillation de monomères dont l'acide acrylique. Ces documents-brevets montrent que l'utilisation du gaz NO₂ n'a pas été envisagée car cette molécule était admise comme pouvant initier des polymérisations.

On peut également citer US-A-4 338 162 qui décrit l'utilisation d'oxydes d'azote, préférentiellement le monoxyde d'azote en absence d'oxygène, durant la distillation d'esters cyanoacryliques.

Il est également possible d'utiliser des molécules générant du NO in situ (par exemple N-phényl N-nitroso-hydroxylamine, N,N-dinitroso phénylènediamine etc.) pour la stabilisation des monomères acryliques (cf EP-A-0 522 709 qui décrit l'utilisation de N',N'-dinitroso phénylènediamine comme molécule générant NO et qui conseille d'ajouter de l'oxygène si l'on travaille en présence d'hydroquinone ; WO-A-97/12851 qui décrit l'utilisation du couple, N-nitroso-phényl hydroxylamine et sel de cuivre pour inhiber la polymérisation des composés vinyliques ; US-A-2 741 583 qui décrit l'utilisation d'un mélange d'oxydes d'azote provenant de l'attaque d'un nitrite par un acide minéral pour stabiliser des esters acryliques).

Sachant que les stabilisants couramment utilisés lors de la purification de l'acide acrylique nécessitent de l'oxygène pour être actifs (composés phénoliques comme l'hydroquinone et son éther méthylique, composés quinoniques comme la benzoquinone) ou présentent une meilleure efficacité en présence d'oxygène (dérivés de phénothiazine, d'amines aromatiques, de thiocarbamates, de sels de métaux de transition et de radicaux libres stables), l'utilisation du gaz NO ne semble alors pas possible dans les conditions actuelles de fonctionnement.

La Société déposante a donc cherché à résoudre ce problème de stabilisation, tout en maintenant une stabilisation conventionnelle de la phase liquide notamment par l'utilisation d'inhibiteurs nécessitant une introduction d'oxygène.

A cet effet, et afin de vérifier l'effet annoncé dans la littérature du NO₂ sur les monomères acryliques, la Société déposante a réalisé des essais de distillation de monomères acryliques en présence de NO₂, ce qui l'a amené à observer que, de façon surprenante, dans certaines conditions opératoires, le gaz NO₂, industriellement intéressant car moins coûteux que le gaz NO, apporte une stabilisation durant la distillation du monomère acrylique, autrement dit joue le rôle d'inhibiteur de la phase gazeuse, alors qu'il est généralement connu comme amorceur de polymérisation.

Les conditions ci-dessus consistent à respecter, d'une part un certain rapport oxygène/vapeur organique - le contrôle de ce paramètre permettant d'optimiser la stabilité de la phase liquide -, et, d'autre part, une certain rapport NO₂/vapeur organique - le contrôle de ce paramètre permettant d'assurer une efficacité inhibitrice du NO₂ en phase gazeuse et d'empêcher l'amorçage de la polymérisation.

L'injection directe de NO₂ permet d'améliorer la stabilité des monomères durant leur distillation, du fait de la possibilité d'introduction du NO₂ directement aux emplacements critiques, non accessibles par du liquide (col de cygne de colonne, tête de colonne, etc.), ce que ne permet pas l'utilisation de molécules générant du NO₂ in situ.

Par ailleurs, cette injection directe offre les avantages complémentaires de la plus grande facilité d'injection d'un gaz par rapport à un liquide dans une colonne à distiller industrielle et de l'amélioration de l'hygiène industrielle par l'injection d'un gaz plutôt que d'un liquide.

La présente invention a donc pour objet un procédé de purification d'un monomère (méth) acrylique choisi parmi les acides (méth)acryliques et leurs esters, par distillation en présence d'au moins un inhibiteur de polymérisation nécessitant une introduction d'oxygène et/ou un inhibiteur présentant une meilleure efficacité en présence d'oxygène en vue de la stabilisation de la phase liquide, caractérisé par le fait que l'on conduit la distillation également en présence de gaz NO₂, avec un rapport (p/p) oxygène/vapeur organique compris entre 0,02 et 3%, en particulier compris entre 0,04 et 0,5%, et avec un rapport (p/p) NO₂/vapeur organique compris entre 0,01 ppm et 50 ppm, en particulier compris entre 1 ppm et 30 ppm.

Conformément à différentes autres caractéristiques particulières de l'invention :
- on conduit la distillation dans une colonne sous une pression de 1,33 x 10³ - 6,66 x 10⁴ Pa (10 à 500 mmHg), avec une température de 60 à 200°C dans le bouilleur et de 40 à 100°C en tête de colonne, le flux de monomère à purifier étant alimente en continu ;
- on effectue l'introduction d'oxygène dans le bouilleur ;
- on effectue l'introduction de NO₂ gazeux dans l'alimentation et/ou dans les emplacements de la colonne non accessibles par du liquide ; le gaz NO₂ peut provenir de l'oxydation de NO, NO pouvant être obtenu par la décomposition de réactifs, comme cela est bien connu de l'homme du métier.
- on purifie un monomère (méth) acrylique choisi parmi l'acide acrylique, l'acide méthacrylique, les acrylates d'alkyle en C₁-C₁₀ et les méthacrylates d'alkyle en C₁-C₁₀, plus particulièrement l'acide acrylique ;
- on utilise, comme inhibiteur de polymérisation nécessitant l'introduction d'oxygène, un inhibiteur phénolique comme l'hydroquinone, l'éther méthylique de l'hydroquinone, l'hydroxytoluène butylé ou le 2,4-diméthyl 6-butyl phénol, et/ou un inhibiteur présentant une meilleure efficacité en présence d'oxygène, comme la phénothiazine et ses dérivés, la paraphénylène diamine et ses dérivés, un thiocarbamate métallique, comme le dibutyldithiocarbamate de cuivre, un carboxylate de métal de transition, comme l'acétate de manganèse, un radical stable nitroxyde, comme le 4-hydroxy tétraméthylpipéridine N-oxyl ; et
- on introduit l'inhibiteur de polymérisation à raison de 50 à 2000 ppm par rapport aux vapeurs organiques condensées en tête de colonne.

Les Exemples suivants illustrent la présente invention sans toutefois en limiter la portée.

### EXEMPLES 1 à 5

### Mode opératoire général

Les exemples présentés ont été réalisés à partir d'un montage en verre simulant une distillation en continu de l'une des étapes de purification de l'acide acrylique.

Ce montage est constitué d'une colonne à distiller équipée de plateaux perforés à déversoir et d'un bouilleur à thermosiphon, et surmontée d'un col de cygne. Les vapeurs organiques sont condensées grâce à un réfrigérant conventionnel. Une partie du liquide condensé est recyclée en tête de colonne après addition de stabilisants de phase liquide. La distillation est réalisée sous pression réduite de 2,66 x 10⁴ Pa (200 mmHg) avec une température de 97°C dans le bouilleur et 87°C en tête de colonne.

La distillation est effectuée pendant 24 heures. Les polymères apparaissent principalement au niveau de deux zones de condensation des vapeurs organiques : la sonde de température en inox de la tête de colonne et la bride de raccord du col de cygne au réfrigérant. En fin d'essai ces polymères sont séchés, puis pesés.

Le flux utilisé pour tous les essais présentés est constitué d'acide acrylique brut dont l'impureté majeure est l'acide acétique à raison de 4% en poids. Ce flux alimente en continu la colonne à distiller avec un débit de 160 g/h. On prélève en tête de colonne 69 g/h de distillat et on soutire 91 g/h en pied. Le débit de vapeur organique dans la colonne est de 335 g/h.

Les vapeurs organiques condensées (335 g/h) sont stabilisées avec une solution d'acide acrylique contenant 2% en poids d'hydroquinone et 0,25% de dibutylthiocarbamate de cuivre à raison de 2,4 g/h. Une fraction de ces vapeurs organiques condensées (266 g/h) est renvoyée en tête de la colonne à distiller.

### EXEMPLE 1

L'introduction d'oxygène est effectuée dans le bouilleur à raison de 0,21 l/h, soit un rapport (p/p) oxygène/vapeur organique de 0,09%. L'introduction de NO₂ gazeux est effectuée dans l'alimentation avec un débit de 1,9 ml/h, ce qui correspond à un rapport (p/p) NO₂ sur vapeur organique de 12 ppm.

Dans ces conditions, le distillat est clair, limpide, et aucun polymère n'est obtenu au niveau des deux zones de condensation de la colonne.

### EXEMPLE 2

Cet exemple a été réalisé dans les mêmes conditions que l'Exemple 1, mais avec un débit de NO₂ gazeux de 0,8 ml/h. Le rapport (p/p) NO₂ sur vapeur organique est donc de 5 ppm.

Dans ces conditions, le distillat est clair, limpide, et la quantité totale de polymère formé au niveau des deux zones de condensation de la colonne est de 0,5 g.

### EXEMPLE 3

Cet exemple a été réalisé dans les mêmes conditions que l'Exemple 1, mais avec un débit de NO₂ gazeux de 3,9 ml/h. Le rapport (p/p) NO₂ sur vapeur organique est donc de 24 ppm.

Dans ces conditions, le distillat est clair, limpide, et la quantité totale de polymère formé au niveau des deux zones de condensation de la colonne est de 3,2 g.

### EXEMPLE 4 (comparatif)

Cet exemple a été réalisé dans les mêmes conditions que l'Exemple 1, mais sans introduction de NO₂.

Dans ces conditions, le distillat est clair, limpide, et la quantité totale de polymère formé au niveau des deux zones de condensation de la colonne est de 8,1 g.

### EXEMPLE 5 (comparatif)

Cet exemple a été réalisé dans les mêmes conditions que l'Exemple 1, mais avec un débit de NO₂ gazeux de 10,1 ml/h. Le rapport (p/p) NO₂ sur vapeur organique est donc de 62 ppm.

Dans ces conditions, on observe la présence de polymères dans le distillat (trouble important) et la quantité totale de polymère formé au niveau des deux zones de condensation de la colonne est de 1,1 g.

## Revendications

1. Procédé de purification d'un monomère (méth)acrylique choisi parmi les acides (méth)acryliques et leurs esters, par distillation en présence d'au moins un inhibiteur de polymérisation nécessitant une introduction d'oxygène et/ou un inhibiteur présentant une meilleure efficacité en présence d'oxygène en vue de la stabilisation de la phase liquide, **caractérisé par le fait que** l'on conduit la distillation également en présence de gaz NO₂, avec un rapport (p/p) oxygène/vapeur organique compris entre 0,02 et 3%, et avec un rapport (p/p) NO₂/vapeur organique compris entre 0,01 et 50 ppm.

2. Procédé selon la revendication 1, **caractérisé par le fait que** l'on conduit la distillation avec un rapport (p/p) oxygène/vapeur organique compris entre 0,04 et 0,5%.

3. Procédé selon l'une des revendications 1 et 2, **caractérisé par le fait que** l'on conduit la distillation avec un rapport (p/p) NO₂/vapeur organique compris entre 1 et 30 ppm.

4. Procédé selon l'une des revendications 1 à 3, **caractérisé par le fait que** l'on conduit la distillation dans une colonne sous une pression de 1,33 x 10³ à 6,66 x 10⁴ Pa (10 à 500 mmHg), avec une température de 60-200°C dans le bouilleur et de 40-100°C en tête de colonne ; le flux de monomère à purifier étant alimenté en continu.

5. Procédé selon la revendication 4, **caractérisé par le fait que** l'on effectue l'introduction d'oxygène dans le bouilleur.

6. Procédé selon l'une des revendications 4 et 5, **caractérisé par le fait que** l'on effectue l'introduction de NO₂ gazeux dans l'alimentation et/ou dans les emplacements de la colonne non accessibles par du liquide.

7. Procédé selon l'une des revendications 1 à 6, **caractérisé par le fait que** l'on purifie un monomère (méth) acrylique choisi parmi l'acide acrylique, l'acide méthacrylique, les acrylates d'alkyle en C₁-C₁₀ et les méthacrylates d'alkyle en C₁-C₁₀.

8. Procédé selon l'une des revendications 1 à 7, **caractérisé par le fait que** l'on utilise, comme inhibiteur de polymérisation nécessitant l'introduction d'oxygène, un inhibiteur phénolique comme l'hydroquinone, l'éther méthylique de l'hydroquinone, l'hydroxytoluène butylé ou le 2,4-diméthyl 6-butyl phénol, et/ou un inhibiteur présentant une meilleure efficacité en présence d'oxygène, comme la phénothiazine et ses dérivés, la paraphénylène diamine et ses dérivés, un thiocarbamate métallique, comme le dibutyldithiocarbamate de cuivre, un carboxylate de métal de transition, comme l'acétate de manganèse, un radical stable nitroxyde, comme le 4-hydroxy tétraméthylpipéridine N-oxyl.

9. Procédé selon l'une des revendications 1 à 8, **caractérisé par le fait que** l'on introduit l'inhibiteur de polymérisation à raison de 50 à 2000 ppm par rapport aux vapeurs organiques condensées en tête de colonne.

## Patentansprüche

1. Verfahren zur Reinigung eines unter (Meth)acrylsäure und Estern davon ausgewählten (Meth)acryl-monomers durch Destillation in Gegenwart mindestens eines Polymerisationsinhibitors, der die Eintragung von Sauerstoff erfordert, und/oder eines Inhibitors mit besserer Wirksamkeit in Gegenwart von Sauerstoff im Hinblick auf die Stabilisierung der flüssigen Phase, **dadurch gekennzeichnet, daß** man die Destillation außerdem in Gegenwart von NO₂-Gas mit einem Gewichtsverhältnis von Sauerstoff zu organischem Dampf zwischen 0,02 und 3% und einem Gewichtsverhältnis von NO₂ zu organischem Dampf zwischen 0,01 und 50 ppm durchführt.

2. Verfahren nach Anspruch 1, **dadurch gekennzeichnet, daß** man die Destillation mit einem Gewichtsverhältnis von Sauerstoff zu organischem Dampf zwischen 0,04 und 0,5% durchführt.

3. Verfahren nach Anspruch 1 oder 2, **dadurch gekennzeichnet, daß** man die Destillation mit einem Gewichtsverhältnis von NO₂ zu organischem Dampf zwischen 1 und 30 ppm durchführt.

4. Verfahren nach einem der Ansprüche 1 bis 3, **dadurch gekennzeichnet, daß** man die Destillation in einer Kolonne unter einem Druck von 1,33 x 10³ bis 6,66 x 10⁴ Pa (10 bis 500 mmHg) bei einer Temperatur von 60-200°C in der Destillationsblase und 40-100°C am Kopf der Kolonne durchführt, wobei der Strom von zu reinigendem Monomer kontinuierlich zugeführt wird.

5. Verfahren nach Anspruch 4, **dadurch gekennzeichnet, daß** man in die Destillationsblase Sauerstoff einträgt.

6. Verfahren nach Anspruch 4 oder 5, **dadurch gekennzeichnet, daß** man gasförmiges NO₂ in die Zuführung und/oder die für Flüssigkeit nicht zugänglichen Stellen der Kolonne einträgt.

7. Verfahren nach einem der Ansprüche 1 bis 6, **dadurch gekennzeichnet, daß** man ein unter Acrylsäure, Methacrylsäure, C₁-C₁₀-Alkylacrylaten und C₁-C₁₀-Alkylmethacrylaten ausgewähltes (Meth)-acrylmonomer reinigt.

8. Verfahren nach einem der Ansprüche 1 bis 7, **dadurch gekennzeichnet, daß** man als Polymerisationsinhibitor, der die Eintragung von Sauerstoff erfordert, einen phenolischen Inhibitor wie Hydrochinon, Hydrochinonmethylether, Butylhydroxytoluol oder 2,4-Dimethyl-6-butylphenol und/oder einen Inhibitor mit besserer Wirksamkeit in Gegenwart von Sauerstoff wie Phenothiazin und Derivate davon, para-Phenylendiamin und Derivate davon, ein Metallthiocarbamat, wie Kupferdibutyldithiocarbamat, ein Übergangsmetallcarboxylat, wie Manganacetat oder ein stabiles Nitroxidradikal, wie 4-Hydroxytetramethylpiperidin-N-oxyl verwendet.

9. Verfahren nach einem der Ansprüche 1 bis 8, **dadurch gekennzeichnet, daß** man den Polymerisationsinhibitor in einer Menge von 50 bis 2000 ppm, bezogen auf die am Kopf der Kolonne kondensierten organischen Dämpfe, einträgt.

## Claims

1. Process for purifying a (meth)acrylic monomer chosen from (meth)acrylic acids and esters thereof, by distillation in the presence of at least one polymerization inhibitor requiring an introduction of oxygen and/or an inhibitor exhibiting better efficacy in the presence of oxygen for the stabilization of the liquid phase, **characterized in that** the distillation is also carried out in the presence of NO₂ gas, with an oxygen/organic vapour ratio (w/w) of between 0.02 and 3%, and with an NO₂/organic vapour ratio (w/w) of between 0.01 and 50 ppm.

2. Process according to Claim 1, **characterized in that** the distillation is carried out with an oxygen/organic vapour ratio (w/w) of between 0.04 and 0.5%.

3. Process according to either of Claims 1 and 2, **characterized in that** the distillation is carried out with an NO₂/organic vapour ratio (w/w) of between 1 and 30 ppm.

4. Process according to one of Claims 1 to 3, **characterized in that** the distillation is carried out in a column at a pressure of 1.33 × 10³ to 6.66 × 10⁴ Pa (10 to 500 mmHg), with a temperature of 60-200°C in the boiler and of 40-100°C at the top of the column, the stream of monomer to be purified being continuously fed.

5. Process according to Claim 4, **characterized in that** oxygen is introduced into the boiler.

6. Process according to either of Claims 4 and 5, **characterized in that** gaseous NO₂ is introduced into the supply and/or into the sites of the column not accessible to liquid.

7. Process according to one of Claims 1 to 6, **characterized in that** a (meth)acrylic monomer chosen from acrylic acid, methacrylic acid, C₁-C₁₀ alkyl acrylates and C₁-C₁₀ alkyl methacrylates, is purified.

8. Process according to one of Claims 1 to 7, **characterized in that** there is used, as polymerization inhibitor requiring the introduction of oxygen, a phenolic inhibitor such as hydroquinone, methyl ether of hydroquinone, butylated hydroxytoluene or 2,4-dimethyl-6-butylphenol, and/or an inhibitor exhibiting better efficacy in the presence of oxygen, such as phenothiazine and derivatives thereof, para-phenylenediamine and derivatives thereof, a metal thiocarbamate, such as copper dibutyldithiocarbamate, a transition metal carboxylate, such as manganese acetate, a stable nitroxide radical such as N-oxyl-4-hydroxytetramethylpiperidine.

9. Process according to one of Claims 1 to 8, **characterized in that** the polymerization inhibitor is introduced in an amount of 50 to 2000 ppm relative to the condensed organic vapours at the top of the column.
